# EUROPÄISCHE PATENTANMELDUNG

(11) **EP 1 398 318 A1**
(43) Veröffentlichungstag der Anmeldung: **17.03.2004**
(21) Anmeldenummer: 03019118.3
(22) Anmeldetag: 23.08.2003
(51) Int. Cl.: C07D 521/00

(54) **Verfahren zur Durchführung chemischer Reaktionen in der Flüssigphase in Gegenwart eines Katalysators und eines 1,3-substituierten Imidazoliumsalzes**

(30) Priorität: 07.09.2002 DE 10241555
(71) Anmelder: BASF AKTIENGESELLSCHAFT, 67056 Ludwigshafen (DE)
(72) Erfinder: Weigl, Hagen, Dr., 68526 Ladenburg (DE); Ebel, Klaus, Dr., 68623 Lampertheim (DE); Böhm, Volker, Dr., 67105 Schifferstadt (DE)

(57) **Zusammenfassung**

Verfahren zur Durchführung chemischer Reaktionen in der Flüssigphase durch Umsetzung der Edukte in Gegenwart
(a) eines Katalysators; und
(b) eines 1,3-substituierten Imidazoliumsalzes der allgemeinen Formel (I) 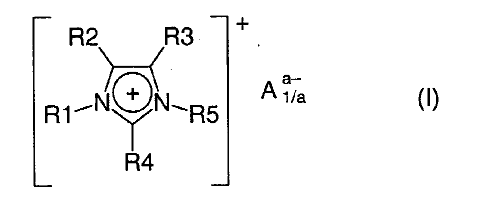 welches einen Schmelzpunkt bei Normaldruck von ≤200°C aufweist,
bei dem man ein 1,3-substituiertes Imidazoliumsalz (I) einsetzt, welches durch
(i) Umsetzung eines 1-substituierten Imidazols der allgemeinen Formel (II) 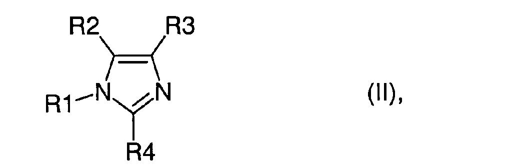 mit einem Kohlensäurediester der allgemeinen Formel (III) 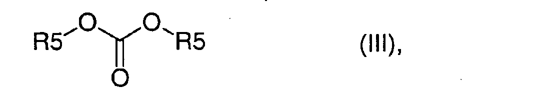 bei einer Temperatur im Bereich von 10 bis 350°C (283 bis 623 K) und einem Druck im Bereich von 0,01 bis 5 MPa abs; und
(ii) Umsetzung des Umsetzungsprodukts aus Schritt (i) mit der gewünschten Protonensäure HₐA (IV) oder deren Salzen bei einer Temperatur im Bereich von -80 bis 200°C (193 bis 473 K) und einem Druck im Bereich von 0,001 bis 1 MPa abs zum 1,3-substituierten Imidazoliumsalz (I);
hergestellt wurde.

## Beschreibung

Die vorliegende Erfindung betrifft ein Verfahren zur Durchführung chemischer Reaktionen in der Flüssigphase durch Umsetzung der Edukte in Gegenwart
(a) eines Katalysators; und
(b) eines 1,3-substituierten Imidazoliumsalzes der allgemeinen Formel (I) 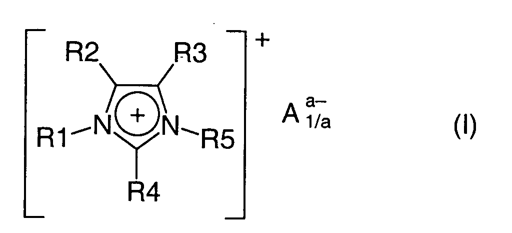 in der
   * die Reste R², R³ und R⁴ unabhängig voneinander Wasserstoff, Halogen oder eine funktionelle Gruppe und die Reste R¹, R², R³, R⁴ und R⁵ unabhängig voneinander einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R⁴ und R¹, R¹ und R² sowie R² und R³ auch miteinander verbunden sein können;
   und
   * A^{a-} für das Anion einer Protonensäure HₐA (IV) steht, wobei a eine ganze, positive Zahl ist und den Ladungszustand des Anions wiedergibt, und A^{a-} für
      Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat;
      tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
      organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
      Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
      (Fluoralkyl)fluorphosphat der allgemeinen Formel (Vd) [PFₓ(C_{y}F_{2y+1-z}H_{z})₆₋ₓ]⁻, wobei 1 ≤x ≤6, 1 ≤y ≤8 und 0 ≤ z ≤ 2y+1; oder
      Imid der allgemeinen Formeln (Ve) [R^{g}-SO₂-N-SO₂-R^{h}]⁻, (Vf) (Rⁱ-SO₂-N-CO-R^{j}]⁻ oder (Vg) [R^{k}-CO-N-CO-R^{l}]⁻, wobei R^{g} bis R¹ unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen; steht,
und welches einen Schmelzpunkt bei Normaldruck von ≤200°C aufweist,
bei dem das 1,3-substituierte Imidazoliumsalz (I) ausgehend von einem 1-substituierten Imidazol und einem Kohlensäurediester hergestellt wurde.

Ferner betrifft die Erfindung die Verwendung des derart hergestellten 1,3-substituierten Imidazoliumsalzes (I), welches einen Schmelzpunkt bei Normaldruck von ≤200°C aufweist, als ionisches Lösungsmittel in der Durchführung chemischer Reaktionen in der Flüssigphase.

Ionische Flüssigkeiten gewinnen zunehmend Bedeutung als Lösungsmittel in der homogenen Übergangsmetallkatalyse. So können durch unterschiedliche Kation/Anion-Kombinationen die Löslichkeitseigenschaften so gezielt eingestellt werden, dass eine systematische Optimierung der homogen katalysierten Reaktionen möglich wird. Eine Übersicht über ionische Flüssigkeiten und deren Einsatz in der homogenen Übergangsmetallkatalyse findet sich in P. Wasserscheid et al., Angew. Chem. 112 (2000), Seite 3926 bis 3945. Darin beschrieben ist unter anderem der Einsatz ionischer Flüssigkeiten als ionisches Lösungsmittel bei homogen katalysierten Hydrierungen, Oxidationen, Hydroformylierungen, Alkoxycarbonylierungen, Heck-Reaktionen, Hydrodimerisierungen, Telomerisierungen, Trost-Tsuji-Kupplungen und Oligomerisierungen.

EP-A 0 748 653 offenbart eine katalytische Zusammensetzung, welche ein quartäres Ammoniumsalz als ionisches, nicht-wässriges Lösungsmittel und eine Verbindung eines Übergangsmetalls der Gruppen 8, 9 und 10 des Periodensystems, enthält und dessen Einsatz in der Hydrierung von Doppel- und Dreifachbindungen ungesättigter Verbindungen.

WO 02/34722 beschreibt den Einsatz von 1,2,3-substituierten, von 1,2,3,4-substituierten und von 1,2,3,4,5-substituierten Imidazoliumsalzen als ionisches Lösungsmittel, insbesondere bei Übergangsmetall katalysierten Reaktionen. Deren Herstellung kann durch zwei verschiedene Methoden erfolgen: (1) Bei der indirekten Route wird das entsprechende, substituierte Imidazol mit einem organischen Halogenid umgesetzt und das Halogenidion des substituierten Imidazolium-halogenids durch Ionenaustausch durch das gewünschte Anion ausgetauscht. (2) Bei der direkten Route wird das entsprechende, substituierte Imidazol mit einem Alkyltriflat oder einem Trialkyloxoniumsalz des gewünschten Anions (z.B. Triethyloxonium-tetrafluoroborat) umgesetzt. Nachteilig an der Herstellung über die indirekte Route (1) ist der hohe Aufwand durch den nachfolgend erforderlichen Ionenaustausch und die gegebenenfalls damit verbundene Extraktion des Produkts mit einem organischen Lösungsmittel sowie der verbleibende Restgehalt an Halogenidionen, welcher die Übergangsmetall katalysierten Reaktionen negativ beeinflussen kann. So weisen beispielsweise P. Dyson et al. in Electrochemical Society Proceedings, Volume 99-41, Seite 161 bis 168 auf Probleme in der Flüssigphasenhydrierung und bei der Suzuki-Reaktion beim Einsatzes von Chlorid-haltigen ionischen Flüssigkeiten hin. Nachteilig an der Herstellung über die direkte Route (2) ist, dass das Alkylierungsreagenz sowohl den Substituenten als auch das Anion festlegt und somit die Variationsbreite und Flexibiltät einschränkt. Zudem sind Trialkyloxoniumsalze nur durch eine relativ aufwändige Herstellung erhältlich, welche deren technischen Einsatz sowohl hinsichtlich der Verfügbarkeit als auch der Wirtschaftlichkeit entgegensteht.

EP-A 0 985 668 offenbart, dass bei der Umsetzung von 1-Methylimidazol mit Dimethylcarbonat entgegen den Erwartungen nicht das 1,3-Dimethylimidazolium-methylcarbonat, sondern das 1,3-Dimethylimidazolium-4-carboxylat in hoher Ausbeute gebildet wird.

EP-A 1 182 196 lehrt die halogenidfreie Herstellung von 1,3-substituierten Imidazoliumsalzen durch Umsetzung des zugrundeliegenden 1-substituierten Imidazols mit dem entsprechenden organischen Disulfat als Alkylierungsagens und anschließendem Ionenaustausch mit einem Metallsalz, welches das gewünschte Anion enthält. So erfolgt beispielsweise die Herstellung von 1-Butyl-3-methyl-imidazolium-tetrafluoroborat durch Umsetzung von 1-Butylimidazol mit Dimethylsulfat, nachfolgender Behandlung mit Natrium-tetrafluoroborat und mehrfache Extraktion des Produkts mit Methylenchlorid. Das genannte Verfahren führt zwar zu anscheinend chloridfreien 1,3-substituierten Imidazoliumsalzen, besitzt aber den entscheidenden Nachteil des Einsatzes von organischem Disulfat, welches aufgrund seiner karzinogenen und ätzenden Wirkung ein erhöhtes Sicherheitsrisiko darstellt und daher einen enormen Sicherheitsaufwand bedingt. Des Weiteren ist der Ionenaustausch durch Zugabe eines Metallsalzes, welches das gewünschte Anion enthält, sehr aufwändig, zumal der Ansatz anschließend extraktiv unter Einsatz eines organischen Lösungsmittels aufzuarbeiten ist.

WO 96/18459 offenbart die Herstellung halogenidfreier ionischer Flüssigkeiten durch Umsetzung eines Halogenids des gewünschten Imidazolium-, Pyridinium- oder Phosphonium-Kations mit einem Bleisalz, dessen Anion das für die ionische Flüssigkeit gewünschte Anion darstellt und Abtrennung des ausgefallenen Bleihalogenids. Nachteilig an dem beschriebenen Verfahren ist der Einsatz stöchiometrischer Mengen an toxischen, im Allgemeinen gut wasserlöslichen Bleisalzen und der Anfall stöchiometrischer Mengen an Bleihalogeniden, welche zu entsorgen beziehungsweise wie-der aufzuarbeiten sind.

EP-A 0 291 074 lehrt die Herstellung von quartären Ammoniumsalzen in einer Zweistufenreaktion durch Umsetzung eines tertiären Amins mit einem Kohlensäurediester unter Bildung eines quartären Ammoniumcarbonats und Vermischen des erhaltenen quartären Ammoniumcarbonats mit einer Säure unter Entfernung von Kohlendioxid und Bildung des quartären Ammoniumsalzes. Nach der Lehre der zitierten Schrift eignen sich die so hergestellten quartären Ammoniumsalze als Katalysatoren, beispielsweise als Phasen-Transfer-Katalysatoren, als Elektrolyte für wässrige oder organische Elektrolytlösungen, als diverse Additive und für den pharmazeutischen Bereich.

Die Aufgabe der vorliegenden Erfindung bestand darin, ein Verfahren zur Durchführung chemischer Reaktionen in der Flüssigphase durch Umsetzung der Edukte in Gegenwart eines Katalysators und eines 1,3-substituierten Imidazoliumsalzes zu finden, welches die oben genannten Nachteile nicht mehr besitzt, bei dem das einzusetzende 1,3-substituierte Imidazoliumsalz in einfacher Art und Weise unter Vermeidung des Einsatzes toxischer, karzinogener und ätzender Einsatzstoffe und ohne aufwändige Reinigung praktisch chlorid-, bromid- und iodidfrei zugänglich ist und welches in der durchzuführenden chemischen Reaktion in der Flüssigphase einen hohen Umsatz, eine hohe Selektivität, eine hohe Ausbeute und eine hohe Raum-Zeit-Ausbeute ermöglicht.

Demgemäß wurde ein Verfahren zur Durchführung chemischer Reaktionen in der Flüssigphase durch Umsetzung der Edukte in Gegenwart
(a) eines Katalysators; und
(b) eines 1,3-substituierten Imidazoliumsalzes der allgemeinen Formel (I) 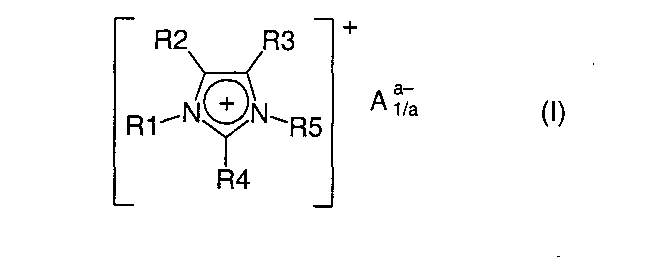 in der
   * die Reste R², R³ und R⁴ unabhängig voneinander Wasserstoff, Halogen oder eine funktionelle Gruppe und die Reste R¹, R², R³, R⁴ und R⁵ unabhängig voneinander einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R⁴ und R¹, R¹ und R² sowie R² und R³ auch miteinander verbunden sein können;
   und
   * A^{a-} für das Anion einer Protonensäure HₐA (IV) steht, wobei a eine ganze, positive Zahl ist und den Ladungszustand des Anions wiedergibt, und A^{a-} für
      Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat;
      tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
      organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
      Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
      (Fluoralkyl)fluorphosphat der allgemeinen Formel (Vd) [PFₓ(C_{y}F_{2y+1-z}H_{z)6-x}]⁻, wobei 1 ≤ x ≤6, 1 ≤ y≤ 8 und 0 ≤ z ≤ 2y+1; oder
      Imid der allgemeinen Formeln (Ve) [R^{g}-SO₂-N-SO₂-R^{h}]⁻, (Vf) [Rⁱ-SO₂-N-CO-R^{j}]⁻ oder (Vg) [R^{k}-CO-N-CO-R¹]⁻, wobei R^{g} bis R¹ unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
      steht,
   und welches einen Schmelzpunkt bei Normaldruck von ≤200°C aufweist,
   gefunden, das dadurch gekennzeichnet ist, dass man ein 1,3-substituiertes Imidazoliumsalz (I) einsetzt, welches durch

(i) Umsetzung eines 1-substituierten Imidazols der allgemeinen Formel (II) 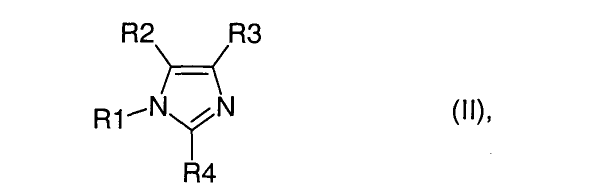 in der die Reste R¹ bis R⁴ die oben genannte Bedeutung besitzen, mit einem Kohlensäurediester der allgemeinen Formel (III) 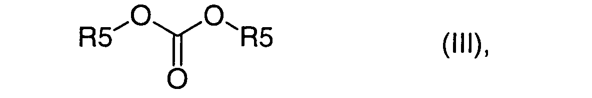 in der der Rest R⁵ die oben genannte Bedeutung besitzt, bei einer Temperatur im Bereich von 10 bis 350°C (283 bis 623 K) und einem Druck im Bereich von 0,01 bis 5 MPa abs; und
(ii) Umsetzung des Umsetzungsprodukts aus Schritt (i) mit der gewünschten Protonensäure HₐA (IV) oder deren Salzen bei einer Temperatur im Bereich von -80 bis 200°C (193 bis 473 K) und einem Druck im Bereich von 0,001 bis 1 MPa abs zum 1,3-substituierten Imidazoliumsalz (I);
hergestellt wurde.

Das beim erfindungsgemäßen Verfahren einzusetzende 1,3-substituierte Imidazoliumsalz (I) enthält das 1,3-substituierte Imidazoliumkation der allgemeinen Formel (Ia) 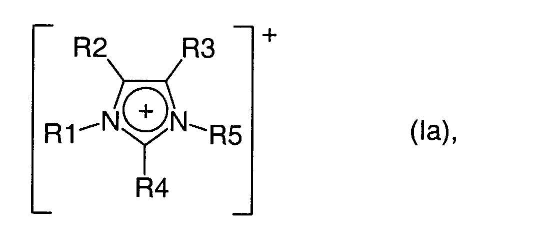 in dem die Reste R², R³ und R⁴ unabhängig voneinander Wasserstoff, Halogen oder eine funktionelle Gruppe und die Reste R¹, R², R³, R⁴ und R⁵ unabhängig voneinander einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R⁴ und R¹, R¹ und R² sowie R² und R³ auch miteinander verbunden sein können.

Als Heteroatome kommen prinzipiell alle Heteroatome in Frage, welche in der Lage sind, formell eine -CH₂-, eine -CH=, eine C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen seien insbesondere -O-, -S-, -SO-, -SO₂-, -NR-, -N=, -PR-, -PR₂ und -SiR₂- genannt, wobei es sich bei den Resten R um den verbleibenden Teil des Kohlenstoff enthaltenden Rests handelt. Der Kohlenstoff enthaltende Rest kann dabei im Falle von R², R³ und R⁴ auch direkt über das Heteroatom an den Imidazoliumring gebunden sein.

Als funktionelle Gruppen kommen prinzipiell alle funktionellen Gruppen in Frage, welche an ein Kohlenstoffatom oder ein Heteroatom gebunden sein können. Als geeignete Beispiele seien -OH, =O (insbesondere als Carbonylgruppe), -NH₂, =NH, -COOH und -CONH₂ genannt. Funktionelle Gruppen und Heteroatome können auch direkt benachbart sein, so dass auch Kombinationen aus mehreren benachbarten Atomen, wie etwa -COO- (Ester), -CONH- (sekundäres Amid) oder -CONR- (tertiäres Amid), mit umfasst sind.

Als Halogene seien Fluor, Chlor, Brom, Iod sowie die Cyanogruppe als Pseudohalogen genannt.

Als einwertige Kohlenstoff enthaltende organische, gesättigte oder ungesättigte, acyclische oder cyclische, aliphatische, aromatische oder araliphatische Reste mit 1 bis 30 Kohlenstoffatomen stehen die Reste R¹ bis R⁵ unabhängig voneinander bevorzugt für
- C₁- bis C₃₀-Alkyl und deren aryl-, heteroaryl-, cycloalkyl-, halogen-, hydroxy-, amino-, carboxy-, formyl-, -O-, -CO-, - CO-O- oder -CO-N< substituierte Komponenten, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl, Triacontyl, Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl, Methoxy, Ethoxy, Formyl, Acetyl oder CₙF_{2(n-a)+(1-b)}H_{2a+b} mit n ≤30, 0 ≤a ≤n und b = 0 oder 1 (beispielsweise CF₃, C₂F₅, CH₂CH₂-C₍ₙ₋₂₎F₂₍ₙ₋₂₎₊₁, C₆F₁₃, C₈F₁₇, C₁₀F₂₁, C₁₂F₂₅);
- C₃- bis C₁₂-Cycloalkyl und deren aryl-, heteroaryl-, cycloalkyl-, halogen-, hydroxy-, amino-, carboxy-, formyl-, -O-, - CO- oder -CO-O-substituierte Komponenten, wie beispielsweise Cyclopentyl, 2-Methyl-1-cyclopentyl, 3-Methyl-1-cyclopentyl, Cyclohexyl, 2-Methyl-1-cyclohexyl, 3-Methyl-1-cyclohexyl, 4-Methyl-1-cyclohexyl oder CₙF_{2(n-a)-(1-b)}H_{2a-b} mit n ≤30, 0 ≤ a ≤ n und b = 0 oder 1;
- C₂- bis C₃₀-Alkenyl und deren aryl-, heteroaryl-, cycloalkyl-, halogen-, hydroxy-, amino-, carboxy-, formyl-, -O-, -CO- oder - CO-O-substituierte Komponenten, wie beispielsweise 2-Propenyl, 3-Butenyl, cis-2-Butenyl, trans-2-Butenyl oder CₙF_{2(n-a)-(1-b)}H_{2a-b} mit n ≤30, 0 ≤a ≤n und b = 0 oder 1;
- C₃- bis C₁₂-Cycloalkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 3-Cyclopentenyl, 2-Cyclohexenyl, 3-Cyclohexenyl, 2,5-Cyclohexadienyl oder CₙF_{2(n-a)-3(1-b)}H_{2a-3b} mit n ≤30, 0 ≤ a ≤ n und b = 0 oder 1; und
- Aryl oder Heteroaryl mit 2 bis 30 Kohlenstoffatomen und deren alkyl-, aryl-, heteroaryl-, cycloalkyl-, halogen-, hydroxy-, amino-, carboxy-, formyl-, -O-, -CO- oder -CO-O-substituierte Komponenten, wie beispielsweise Phenyl, 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl oder C₆F(5-a)Hₐ mit 0≤ a ≤ 5.

Als miteinander verbundene Kohlenstoff enthaltenden organische, gesättigte oder ungesättigte, acyclische oder cyclische, aliphatische, aromatische oder araliphatische Reste mit 1 bis 30 Kohlenstoffatomen stehen die Reste R⁴ mit R¹, R¹ mit R² und R² mit R³ unabhängig voneinander bevorzugt für
- einen unverzweigten oder verzweigten, acyclischen oder cyclischen, unsubstituierten oder substituierten, gesättigten Rest mit 3 bis 10 Atomen in der Kette, bei dem CH₂-Gruppen auch durch Heterogruppen, wie beispielsweise -CO-, -O- oder -NRersetzt sein können und bei dem ein oder mehrere der Wasserstoffatome durch Substituenten, wie beispielsweise Alkyl-, Arylgruppen, funktionelle Gruppen oder Halogen ersetzt sein können, wie beispielsweise -CH₂CH₂CH₂-, -CH₂CH₂CH₂CH₂-, - CH₂CH₂CH₂CH₂CH₂-;
- einen unverzweigten oder verzweigten, acyclischen oder cyclischen, unsubstituierten oder substituierten, ungesättigten Rest mit 3 bis 10 Atomen in der Kette und eine oder mehrere Doppelbindungen, bei dem CH₂-Gruppen auch durch Heterogruppen, wie beispielsweise -CO-, -O- oder -NR- und CH-Gruppen auch durch Heterogruppen, wie beispielsweise -N= ersetzt sein können und bei dem ein oder mehrere der Wasserstoffatome durch Substituenten, wie beispielsweise Alkyl-, Arylgruppen, funktionelle Gruppen oder Halogen ersetzt sein können, wie beispielsweise -CH=CH-CH=CH-.

Besonders bevorzugt stehen die Reste R¹ und R⁵ unabhängig voneinander für unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl oder für 6-Hydroxyhexyl.

Ganz besonders bevorzugt steht der Reste R¹ für Methyl, Ethyl, 1-Propyl, 2-Propyl, 1-Butyl, 1-Hexyl, Octyl, Decyl, Dodecyl, Trifluormethyl, Pentafluorethyl, Heptafluorpropyl oder 6-Hydroxyhexyl.

Ganz besonders bevorzugt steht der Reste R⁵ für Methyl und Ethyl.

Besonders bevorzugt stehen die Reste R², R³ und R⁴ unabhängig voneinander für Wasserstoff, Fluor, Chlor, Brom, Phenyl, C₆F₍₅₋ₐ₎Hₐ mit 0 ≤ a ≤5 oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl.

Ganz besonders bevorzugt stehen die Reste R², R³ und R⁴ unabhängig voneinander für Wasserstoff, Fluor, Chlor, Brom, Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl oder 3,3-Dimethyl-2-butyl.

Ganz besonders bevorzugt setzt man beim erfindungsgemäßen Verfahren ein 1,3-substituiertes Imidazoliumsalz (I) ein, bei dem die Reste R², R³ und R⁴ unabhängig voneinander Wasserstoff, Fluor, Chlor, Brom, Phenyl, C₆F₍₅₋ₐ₎Hₐ mit 0 ≤ a ≤5 oder eine unverzweigte oder verzweigte C₁- bis C₁₂-Alkylgruppe und die Reste R¹ und R⁵ unabhängig voneinander eine unverzweigte oder verzweigte C₁- bis C₁₂-Alkylgruppe bedeuten.

Insbesondere setzt man beim erfindungsgemäßen Verfahren ein 1,3-substituierte Imidazoliumsalz (I) ein, welches als 1,3-substituierte Imidazoliumkation 1,3-Dimethylimdazolium, 1-Ethyl-3-methylimidazolium, 1-Methyl-3-propylimidazolium, 1-Isopropyl-3-methylimidazolium, 1-Butyl-3-methylimidazolium, 1-Methyl-3-pentylimidazolium, 1-Hexyl-3-methylimidazolium, 1-Heptyl-3-methylimidazolium, 1-Methyl-3-octylimidazolium oder 1-Ethylhexyl-3-methylimidazolium enthält.

Das beim erfindungsgemäßen Verfahren einzusetzende 1,3-substituierte Imidazoliumsalz (I) enthält das Anion A^{a-}, wobei dieses für das Anion einer Protonensäure HₐA (IV) steht, a eine ganze, positive Zahl ist und den Ladungszustand des Anions wiedergibt, und A^{a-} für

Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat; tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;

Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
(Fluoralkyl)fluorphosphat der allgemeinen Formel (Vd) [PFₓ(C_{y}F_{2y+1-z}H_{z})₆₋ₓ]⁻, wobei 1 ≤x ≤6, 1 ≤y ≤8 und 0 ≤z ≤ 2y+1; oder

Imid der allgemeinen Formeln (Ve) [R^{g}-SO₂-N-SO₂-R^{h}]⁻, (Vf) [Rⁱ-SO₂-N-CO-R^{j}]⁻ oder (Vg) [R^{k}-CO-N-CO-R¹]⁻, wobei R^{g} bis R¹ unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
steht.

Die Ladung "a-" des Anions A^{a-} beträgt "1-", "2-" oder "3-". Als Beispiele zweifach negativ geladener Anionen seien Sulfat, Hydrogenphosphat und Carbonat genannt. Als Beispiel eines dreifach negativ geladenen Anions sei Phosphat genannt.

Als Heteroatome kommen prinzipiell alle Heteroatome in Frage, welche in der Lage sind, formell eine -CH₂-, eine -CH=, eine C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen seien insbesondere -O-, -S-, -SO-, -SO₂-, -NR-, -N=, -PR-, -PR₂ und -SiR₂- genannt, wobei es sich bei den Resten R um den verbleibenden Teil des Kohlenstoff enthaltenden Rests handelt. Der Kohlenstoff enthaltende Rest kann dabei im Falle von R², R³ und R⁴ auch direkt über das Heteroatom an den Imidazoliumring gebunden sein.

Als funktionelle Gruppen kommen prinzipiell alle funktionellen Gruppen in Frage, welche an ein Kohlenstoffatom oder ein Heteroatom gebunden sein können. Als geeignete Beispiele seien -OH, =O (insbesondere als Carbonylgruppe), -NH₂, =NH, -COOH und -CONH₂ genannt. Fuktionelle Gruppen und Heteroatome können auch direkt benachbart sein, so dass auch Kombinationen aus mehreren benachbarten Atomen, wie etwa -COO- (Ester), -CONH- (sekundäres Amid) oder -CONR- (tertiäres Amid), mit umfasst sind.

Als Halogene seien Fluor, Chlor, Brom, Iod sowie die Cyanogruppe als Pseudohalogen genannt.

Als Kohlenstoff enthaltende organische, gesättigte oder ungesättigte, acyclische oder cyclische, aliphatische, aromatische oder araliphatische Reste mit 1 bis 30 Kohlenstoffatomen stehen die Reste R^{a} bis R^{d} beim tetrasubstituiertes Borat (Va), der Rest R^{e} beim organischen Sulfonat (Vb), der Rest R^{f} beim Carboxylat (Vc) und die Reste R^{g} bis R¹ bei den Imiden (Ve), (Vf) und (Vg) unabhängig voneinander bevorzugt für
- C₁- bis C₃₀-Alkyl und deren aryl-, heteroaryl-, cycloalkyl-, halogen-, hydroxy-, amino-, carboxy-, formyl-, -O-, -CO-, - CO-O- oder -CO-N< substituierte Komponenten, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl, Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl, Methoxy, Ethoxy, Formyl, Acetyl oder CₙF_{2(n-a)+(1-b)}H_{2a+b} mit n ≤30, 0 ≤ a ≤ n und b = 0 oder 1 (beispielsweise CF₃, C₂F₅, CH₂CH₂-C₍ₙ₋₂₎F₂₍ₙ₋₂₎₊₁, C₆F₁₃, C₈F₁₇, C₁₀F₂₁, C₁₂F₂₅);
- C₃- bis C₁₂-Cycloalkyl und deren aryl-, heteroaryl-, cycloalkyl-, halogen-, hydroxy-, amino-, carboxy-, formyl-, -O-, -CO- oder -CO-O-substituierte Komponenten, wie beispielsweise Cyclopentyl, 2-Methyl-1-cyclopentyl, 3-Methyl-1-cyclopentyl, Cyclohexyl, 2-Methyl-1-cyclohexyl, 3-Methyl-1-cyclohexyl, 4-Methyl-1-cyclohexyl oder CₙF_{2(n-a)-(1-b)}H_{2a-b} mit n ≤30, 0 ≤ a ≤ n und b = 0 oder 1;
- C₂- bis C₃₀-Alkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 2-Propenyl, 3-Butenyl, cis-2-Butenyl, trans-2-Butenyl oder CₙF_{2(n-a)-(1-b})H_{2a-b} mit n ≤30, 0 ≤ a ≤ n und b = 0 oder 1;
- C₃- bis C₁₂-Cycloalkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 3-Cyclopentenyl, 2-Cyclohexenyl, 3-Cyclohexenyl, 2,5-Cyclohexadienyl oder CₙF_{2(n-a)-3(1-b)}H_{2a-3b} mi t n ≤30, 0 ≤ a ≤ n und b = 0 oder 1; und
- Aryl oder Heteroaryl mit 2 bis 30 Kohlenstoffatomen und deren alkyl-, aryl-, heteroaryl-, cycloalkyl-, halogen-, hydroxy-, amino-, carboxy-, formyl-, -O-, -CO- oder -CO-O-substituierte Komponenten, wie beispielsweise Phenyl, 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl oder C₆F₍₅₋ₐ₎Hₐ mit 0 ≤a ≤5.

Handelt es sich bei dem Anion A^{a-} um ein tetrasubstituiertes Borat (Va) [BR^{a}R^{b}R^{c}R^{d}]⁻, so sind bei diesem bevorzugt alle vier Reste R^{a} bis R^{d} identisch, wobei diese bevorzugt für Fluor, Trifluormethyl, Pentafluorethyl, Phenyl, 3,5-Bis(trifluormethyl)phenyl stehen. Besonders bevorzugte tetrasubstituierte Borate (Va) sind Tetrafluoroborat, Tetraphenylborat und Tetra[3,5-bis(trifluormethyl)phenyl]borat.

Handelt es sich bei dem Anion A^{a-} um ein organisches Sulfonat (Vb) [R^{e}-SO₃]⁻, so steht der Rest R^{e} bevorzugt für Methyl, Trifluormethyl, Pentafluorethyl, p-Tolyl oder C₉F₁₉. Besonders bevorzugte organische Sulfonate (Vb) sind Trifluormethansulfonat (Triflat), Methansulfonat und Nonadecafluorononansulfonat (Nonaflat).

Handelt es sich bei dem Anion A^{a-} um ein Carboxylat (Vc) [R^{f}-COO]⁻, so steht der Rest R^{f} bevorzugt für Wasserstoff, Trifluormethyl, Pentafluorethyl, Phenyl, Hydroxy-phenyl-methyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Carboxylate (Vc) sind Formiat, Acetat, Propionat, Butyrat, Valeriat, Benzoat, Mandelat, Trichloracetat, Dichloracetat, Chloracetat, Trifluoracetat, Difluoracetat, Fluoracetat.

Handelt es sich bei dem Anion A^{a-} um ein (Fluoralkyl)fluorphosphat (Vd) [PFₓ(C_{y}F_{2y+1-z}H_{z})₆₋ₓ]⁻, so ist z bevorzugt 0. Besonders bevorzugt sind (Fluoralkyl)fluorphosphate (Vd), bei denen z = 0, x = 3 und 1 ≤ y ≤ 4, konkret [PF₃(CF₃)₃]⁻, [PF₃(C₂F₅)₃]⁻, [PF₃(C₃F₇)₃]⁻ und [PF₃(C₄F₇)₃]⁻.

Handelt es sich bei dem Anion A^{a-} um ein Imid (Ve) [R^{g}-SO₂-N-SO₂-R^{h}]⁻, (Vf) [Rⁱ-SO₂-N-CO-R^{j}]⁻ oder (Vg) (R^{k}-CO-N-CO-R¹]⁻, so stehen die Reste R^{g} bis R¹ unabhängig voneinander bevorzugt für Wasserstoff, Trifluormethyl, Pentafluorethyl, Phenyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Imide (Ve), (Vf) und (Vg) sind [F₃C-SO₂-N-SO₂-CF₃]⁻, [F₃C-SO₂-N-CO-CF₃]⁻, [F₃C-CO-N-CO-CF₃]⁻ und jene, in denen die Reste R^{g} bis R¹ unabhängig voneinander für Wasserstoff, Methyl, Ethyl, Propyl, Butyl, Phenyl, Trichlormethyl, Dichlormethyl, Chlormethyl, Trifluormethyl, Difluormethyl oder Fluormethyl stehen.

Ganz besonders bevorzugt setzt man beim erfindungsgemäßen Verfahren ein 1,3-substituierte Imidazoliumsalz (I) ein, welches als Anion A^{a-} Tetrafluoroborat, Hexafluorophosphat, Trifluormethansulfonat, Methansulfonat, Formiat, Acetat, Mandelat, Nitrat, Nitrit oder Trifluoracetat enthält.

Das beim erfindungsgemäßen Verfahren einzusetzende 1,3-substituierte Imidazoliumsalz (I) weist einen Schmelzpunkt von ≤200°C, bevorzugt von ≤150°C, besonders bevorzugt von ≤120°C und ganz besonders bevorzugt von ≤100°C auf.

Ganz besonders bevorzugt werden beim erfindungsgemäßen Verfahren als 1,3-substituierte Imidazoliumsalze (I) jene Salze eingesetzt, welche als Kation 1,3-Dimethylimdazolium, 1-Ethyl-3-methylimidazolium, 1-Methyl-3-propylimidazolium, 1-Isopropyl-3-methylimidazolium, 1-Butyl-3-methylimidazolium, 1-Methyl-3-pentylimidazolium, 1-Hexyl-3-methylimidazolium, 1-Heptyl-3-methylimidazolium, 1-Methyl-3-octylimidazolium, 1-Ethylhexyl-3-methylimidazolium und als Anion Tetrafluoroborat, Hexafluorophosphat, Trifluormethansulfonat, Methansulfonat, Formiat, Acetat, Mandelat, Nitrat, Nitrit, Trifluoracetat enthalten.

Das erfindungsgemäße Verfahren ist insbesondere dadurch charakterisiert, dass das einzusetzende 1,3-substituierte Imidazoliumsalz ( I ) durch
(i) Umsetzung eines 1-substituierten Imidazols der allgemeinen Formel (II) 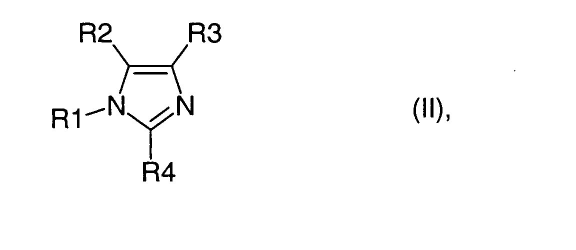 in der die Reste R¹ bis R⁴ die oben genannte Bedeutung besitzen, mit einem Kohlensäurediester der allgemeinen Formel (III) 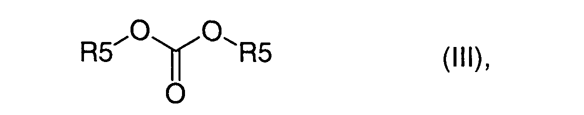 in der der Rest R⁵ die oben genannte Bedeutung besitzt, bei einer Temperatur im Bereich von 10 bis 350°C (283 bis 623 K) und einem Druck im Bereich von 0,01 bis 5 MPa abs; und
(ii) Umsetzung des Umsetzungsprodukts aus Schritt (i) mit der gewünschten Protonensäure HₐA (IV) oder deren Salzen bei einer Temperatur im Bereich von -80 bis 200°C (193 bis 473 K) und einem Druck im Bereich von 0,001 bis 1 MPa abs zum 1,3-substituierten Imidazoliumsalz (I);
hergestellt wurde. Im Folgenden sind die beiden Schritte (i) und (ii) näher erläutert.

### Schritt (i)

Bei der Umsetzung in Schritt (i) beträgt das Molverhältnis zwischen dem 1-substituierten Imidazol (II) und dem Kohlensäurediester (III) im Allgemeinen 0,2 bis 10, bevorzugt 0,5 bis 5 und besonders bevorzugt 0,5 bis 2. Die Temperatur beträgt bei der Umsetzung 10 bis 350°C (283 bis 623 K), bevorzugt 20 bis 250°C und besonders bevorzugt 30 bis 200°C. Der Druck beträgt bei der Umsetzung 0,01 bis 5 MPa abs und bevorzugt 0,05 bis 2,5 MPa abs.

Die Umsetzung in Schritt (i) kann in Gegenwart oder Abwesenheit eines Katalysators erfolgen. Werden Katalysatoren eingesetzt, so sind basische Katalysatoren, wie etwa Natriumcarbonat, Kaliumcarbonat, Natriumhydroxid, Kaliumhydroxid, gegebenenfalls in Gegenwart von Phasentransferkatalysatoren, wie zum Beispiel Tetraalkylammoniumsalze oder Kronenether (z.B. 18-Krone-6), bevorzugt.

Die Umsetzung in Schritt (i) kann in Gegenwart oder Abwesenheit eines inerten Lösungsmittels erfolgen. Unter einem inerten Lösungsmittel sind Lösungsmittel zu verstehen, welche gegenüber den eingesetzten Edukten chemisch inert sind und sich unter den gewählten Bedingungen nicht mit den genannten Stoffen chemisch umsetzen. Als geeignete Lösungsmittel seien aliphatische oder aromatische Kohlenwasserstoffe (z.B. Toluol), Alkohole (z.B. Methanol, Ethanol) oder Ether genannt.

Als Reaktionsapparate können prinzipiell alle Reaktionsapparate eingesetzt werden, welche für Umsetzungen in der Flüssigphase geeignet sind. Als Beispiele seien Rührkessel, Rohrreaktoren oder Reaktionskolonnen genannt. Die Umsetzung kann diskontinuierlich, halbkontinuierlich oder kontinuierlich durchgeführt werden. Dabei können die einzelnen Komponenten in der Regel in beliebiger Reihenfolge zugegeben werden. Wird die Umsetzung kontinuierlich durchgeführt, so ist es jedoch im Allgemeinen zweckmäßig, die einzelnen Komponenten ebenfalls kontinuierlich zuzuführen. Wird die Umsetzung diskontinuierlich durchgeführt, so fügt man die einzelnen Komponenten im Allgemeinen zusammen und bringt sie anschließend auf die gewünschten Reaktionsbedingungen. Es ist aber auch möglich, einzelne Komponenten erst unter den gewünschten Reaktionsbedingungen zuzugeben.

Beim einzusetzenden 1-substituierten Imidazol (II) besitzen die Reste R¹ bis R⁴ die unter (I) genannte Bedeutung. Die einzusetzenden 1-substituierten Imidazole (II) sind durch die üblichen, dem Fachmann bekannten Verfahren erhältlich. Eine Übersicht geeigneter Herstellungsverfahren ist beispielsweise in Ullmann's Encyclopedia of Industrial Chemistry, 6^{th} edition, 2000 electronic release, Chapter "IMIDAZOLE AND DERIVATIVES - Production" gegeben. Besonders bevorzugt werden 1-Methylimdazol, 1-Ethylimidazol, 1-Propylimidazol, 1-Isopropylimidazol, 1-Butylimidazol, 1-Pentylimidazol, 1-Hexyllimidazol, 1-Heptylimidazol, 1-Octylimidazol und 1-(2-Ethylhexyl)-imidazol eingesetzt.

Beim einzusetzenden Kohlensäurediester (III) besitzt der Rest R⁵ die unter (I) genannte Bedeutung. Die einzusetzenden Kohlensäurediester (III) sind durch die üblichen, dem Fachmann bekannten Verfahren erhältlich. Eine Übersicht geeigneter Herstellungsverfahren ist beispielsweise in Ullmann's Encyclopedia of Industrial Chemistry, 6^{th} edition, 2000 electronic release, Chapter "CARBONIC ESTERS - Production" gegeben. Besonders bevorzugt werden Dimethylcarbonat und Dimethylcarbonat eingesetzt.

Das bei Schritt (i) erhaltene Reaktionsgemisch kann direkt zur Umsetzung in Schritt (ii) eingesetzt werden. Es ist aber auch möglich und gegebenenfalls vorteilhaft das erhaltene Reaktionsgemisch durch diverse Schritte aufzuarbeiten. Als mögliche Aufarbeitungsschritte seien die Abtrennung von Feststoffen (beispielsweise durch Filtration oder Zentrifugation), die Abdestillation von Lösungsmittel oder die ionenselektive Membranfiltration genannt.

### Schritt (ii)

Bei der Umsetzung in Schritt (ii) beträgt das Molverhältnis zwischen der gewünschten Protonensäure HₐA (IV) oder deren Salzen und dem in Schritt (i) eingesetztem 1-substituierten Imidazol (II) im Allgemeinen ¹/ₐ · 0,2 bis ¹/ₐ · 5 und bevorzugt ¹/ₐ · 0,5 bis ¹/a · 2, wobei a wie oben definiert die Ladung des Anions A^{a-} ist. Die Temperatur beträgt bei der Umsetzung -80 bis 200°C (193 bis 473 K), bevorzugt -20 bis 150°C (253 bis 423 K) und besonders bevorzugt 0 bis 100°C (273 bis 373 K). Besonders bevorzugt erfolgt die Umsetzung in Schritt (ii) bei einer Temperatur zwischen dem Erstarrungspunkt des 1,3-substituierten Imidazoliumsalzes (I) und dem Siedepunkt des Reaktionsgemischs, wobei dieser über den anliegenden Druck in einem weiten Bereich eingestellt werden kann. Der Druck beträgt bei der Umsetzung 0,001 bis 1 MPa abs und bevorzugt 0,01 bis 0,2 MPa abs.

Die Umsetzung in Schritt (ii) kann in Gegenwart oder Abwesenheit eines inerten Lösungsmittels erfolgen. Dabei ist es prinzipiell auch möglich, ein anderes Lösungsmittel als in Schritt (i) einzusetzen, was jedoch eine vorherige Abtrennung des ersten Lösungsmittels erfordert. Ebenso ist es auch möglich, Schritt (i) ohne Lösungsmittel durchzuführen und erst in Schritt (ii) ein Lösungsmittel zuzugeben.

Die einzusetzende Protonensäure HₐA (IV) oder deren Salz wird im Allgemeinen in Form ihrer wässrigen Lösungen zugegeben, so dass üblicherweise ein zweiphasiges Reaktionsgemisch erhalten wird.
Als Kationen der zuzugebenden Salze sind prinzipiell alle Kationen geeignet, welche zu einem in Wasser im gewünschten Umfang löslichen Salz führen und bei der Isolierung des 1,3-substituierten Imidazoliumsalzes nicht störend wirken. Als Beispiele geeigneter Kationen seien insbesondere Ammonium, Lithium, Natrium, Kalium, Rubidium, Cäsium und Kalzium genannt. Ganz besonders bevorzugt sind Lithium, Natrium und Kalium.

Das bei der Umsetzung freiwerdende Kohlendioxid entweicht aus dem Reaktionsgemisch und wird im Allgemeinen abgeführt.

Als Reaktionsapparate können prinzipiell alle Reaktionsapparate eingesetzt werden, welche für Umsetzungen in der Flüssigphase geeignet sind. Sie sind bereits bei Schritt (i) beschrieben. Die Umsetzung kann diskontinuierlich, halbkontinuierlich oder kontinuierlich durchgeführt werden. Dabei können die einzelnen Komponenten in der Regel in beliebiger Reihenfolge zugegeben werden. Wird die Umsetzung kontinuierlich durchgeführt, so ist es jedoch im Allgemeinen zweckmäßig, die einzelnen Komponenten ebenfalls kontinuierlich zuzuführen. Wird die Umsetzung diskontinuierlich durchgeführt, so fügt man die einzelnen Komponenten im Allgemeinen zusammen und bringt sie anschließend auf die gewünschten Reaktionsbedingungen. Es ist aber auch möglich, einzelne Komponenten erst unter den gewünschten Reaktionsbedingungen zuzugeben. Des Weiteren ist es auch möglich und gegebenenfalls vorteilhaft, die Protonensäure HₐA (IV) unter den Reaktionsbedingungen zum Reaktionsgemisch aus Schritt (i) über einen gewissen Zeitraum von wenigen Sekunden bis mehreren Stunden zulaufen zu lassen.

Das bei Schritt (ii) erhaltene, üblicherweise zweiphasige Reaktionsgemisch wird im Allgemeinen zur Isolation des 1,3-substituierten Imidazoliumsalzes (I) aufgearbeitet. Im Allgemeinen erfolgt hierzu eine Trennung der einzelnen Phasen sowie eine Extraktion zur weiteren Abreicherung von Verunreinigungen. Befindet sich das 1,3-substituierte Imidazoliumsalz (I) in der eher polaren Phase, so extrahiert man bevorzugt mit einem eher unpolaren Lösungsmittel und umgekehrt. Gegebenenfalls ist es auch möglich, die das 1,3-substituierte Imidazoliumsalz (I) enthaltende Phase zu filtrieren, säulenchromatographisch zu reinigen und/oder mit Aktivkohle zu behandeln und/oder mit einem Trockenmittel zu trocknen. Die Isolation des 1,3-substituierten Imidazoliumsalzes (I) erfolgt dann beispielsweise durch Kristallisation, durch Schuppung oder durch Abdestillation des gegebenenfalls noch vorhandenen Lösungsmittels.

Die einzusetzenden Protonensäuren HₐA (IV) oder deren Salze sind durch die üblichen, dem Fachmann bekannten Verfahren erhältlich. Eine Übersicht geeigneter Verfahren zur Herstellung der organischen Sulfonate (Vb) und deren Säuren findet sich beispielsweise in Ullmann's Encyclopedia of Industrial Chemistry, 6^{th} edition, 2000 electronic release, Chapter "SULFONIC ACIDS, ALIPHATIC - Production" und "BENZENESULFONIC ACIDS AND THEIR DERIVATIVES - General Aspects - Production". Eine Übersicht geeigneter Verfahren zur Herstellung der (Fluoralkyl)fluorphosphate (Vd) findet sich beispielsweise in EP-A 1 162 204. Eine Übersicht geeigneter Verfahren zur Herstellung der Imide (Ve) und (Vf) ist beispielsweise in H. Matsumoto et al., Chem. Comm., 2002, Seiten 1726 bis 1727 und EP-A 1 182 196 gegeben.

Das erfindungsgemäße Verfahren zur Durchführung chemischer Reaktionen in der Flüssigphase durch Umsetzung der Edukte wird in Gegenwart eines Katalysators durchgeführt. Unter dem Begriff Katalysator sind alle homogenen und heterogenen Stoffe zu verstehen, welche die Aktivierungsenergie zum Ablauf einer bestimmten chemischen Reaktion herabsetzen und dadurch die Reaktionsgeschwindigkeit erhöhen beziehungsweise die bestimmte chemische Reaktion erst ermöglichen, ohne im Endprodukt der chemischen Reaktion zu erscheinen. Die Katalysatoren können dabei im Reaktionsgemisch beispielsweise homogen gelöst, suspendiert oder heterogen als Feststoff vorliegen. Gegebenenfalls können die einzusetzenden Katalysatoren auch auf einem sogenannten Trägermaterial vorliegen. Als geeignete Trägermaterialien seien beispielsweise metallische Oxide oder Polymere genannt.

Bevorzugt setzt man beim erfindungsgemäßen Verfahren als Katalysator
(i) ein Metall der 3. bis 13. Gruppe des Periodensystems und/oder dessen Verbindungen, oder
(ii) einen Biokatalysator oder ein Biokatalysator enthaltendes System
ein.

Als bevorzugtes Metall der 3. bis 13. Gruppe des Periodensystems und/oder dessen Verbindungen seien Ruthenium, Osmium, Cobalt, Rhodium, Iridium, Nickel, Palladium, Platin, Titan, Zirkon, Vanadium, Mangan, Scandium, Chrom, Molybdän, Wolfram, Eisen, Kupfer, Silber, Aluminium und/oder deren Verbindungen genannt. Besonders bevorzugt setzt man als Katalysator einen Ruthenium, Osmium, Cobalt, Rhodium, Iridium, Nickel, Palladium, Platin, Titan, Zirkon, Vanadium, Mangan und/oder Scandium enthaltenden Übergangsmetall-Komplex ein.

Als Biokatalysatoren seien alle möglichen Stoffe, wie beispielsweise Mikroorganismen, Wirkstoffe, Ergine oder Ergone, zu verstehen, die in Organismen aufgebaut werden oder darin vorkommen und welche in irgendeiner Art und Weise in der Lage sind, chemische Reaktionen zu katalysieren. Als Beispiele von Biokatalysatoren, welche für das erfindungsgemäße Verfahren prinzipiell geeignet sind, seien insbesondere Mikroorganismen, Enzyme, katalytisch aktive Antikörper und RNA-Fragmente genannt. Übersichten über die Durchführung biokatalysierter chemischer Umsetzungen in ionischen Flüssigkeiten finden sich beispielsweise in M. Erbeldinger et al., Biotechnol. Prog. 16, 2000, Seite 1129, in R.M. Lau et al., Org. Lett. 2, 2000, Seite 4189 bis 4191 oder in S.H. Schöfer et al., Chem. Commun., 2001, Seite 425 bis 426.

Beim erfindungsgemäßen Verfahren führt man als chemische Reaktion bevorzugt eine Hydrierung, eine Oxidation, eine Hydroformylierung, eine Heck-Reaktion, eine Hydrodimerisierung, eine Telomerisation, eine Trost-Tsuji-Kupplung, eine Suzuki-Kupplung, eine Kumada-Kupplung, eine Negishi-Kupplung, eine Stille-Kupplung, eine Sonogashira-Reaktion, eine Carbonylierung, eine Isomerisierung, eine Umlagerung, eine Diels-Alder-Reaktion, eine Metathese-Reaktion, eine 1,4-Funktionalisierung von 1,3-Dienen, eine Hydrocyanierung, eine Oligomerisation oder eine Polymerisation durch. Eine allgemeine Übersicht zu den genannten chemischen Reaktionen findet sich in Applied Homogeneous Catalysis with Organometallic Compounds, (B. Cornils, W.A. Herrmann, Eds.), 2^{nd} ed., Wiley-VCH, Weinheim, 2002.

Die folgenden Absätze enthalten eine Übersicht über die einzelnen, bevorzugten chemischen Reaktionen des erfindungsgemäßen Verfahrens. Allen chemischen Reaktion gemeinsam ist, dass sie in flüssigem 1,3-substituierten Imidazoliumsalz (I) durchgeführt werden.

Bei der Hydrierung werden ungesättigte Verbindungen, wie beispielsweise Alkene, Alkine, aromatische Verbindungen, Aldehyde, Imine oder Ketone, durch Umsetzung mit Wasserstoff oder einem anderen Reduktionsmittel (beispielsweise LiAlH₄, NaBH₄ oder iso-Propanol) an einem geeigneten Katalysator zu den korrespondierenden gesättigten oder zumindest weniger ungesättigten Verbindungen hydriert. Als geeignete Katalysatoren seien beispielhaft Verbindungen von Ruthenium, Cobalt, Rhodium, Iridium, Palladium und Platin genannt. Die Hydrierung erfolgt im Allgemeinen bei einer Temperatur von 0 bis 200°C und einem Druck von Atmosphärendruck bis 32,5 MPa abs. Die Hydrierung ungesättigter Verbindungen in Gegenwart einer ionischen Flüssigkeit ist beispielsweise in EP-A 0 748 653 beschrieben.

Bei der Oxidation werden oxidierbare Verbindungen, wie beispielsweise Alkane, Olefine, Alkine, Alkohole, Aldehyde an einem geeigneten Katalysator mit Sauerstoff oder Peroxiden zu Epoxiden, Alkoholen, Carbonsäuren, Carbonsäureanhydriden, Aldehyden, Ketonen, Phenolen, Kohlenmonoxid oder Kohlendioxid umgesetzt. Als geeignete Katalysatoren seien beispielhaft Verbindungen des Vanadiums, Chroms, Molybdäns, Wolframs und Mangans genannt. Die Oxidation erfolgt im Allgemeinen bei einer Temperatur von 0 bis 200°C und einem Druck von Atmosphärendruck bis 32,5 MPa abs.

Bei der Hydroformylierung werden Olefine an einem geeigneten Katalysator mit Wasserstoff und Kohlenmonoxid zu Aldehyden oder, in Kombination mit einer Hydrierung als sogenannte Eintopfreaktion, zu Alkoholen umgesetzt. Als geeignete Katalysatoren seien beispielhaft Verbindungen des Kobalts, Rhodiums, Platins, Palladiums, Rutheniums und Platin/Zinns genannt. Die Hydroformylierung erfolgt im Allgemeinen bei einer Temperatur von 0 bis 200°C und einem Druck von Atmosphärendruck bis 70 MPa abs. Die Hydroformylierung von Olefinen in Gegenwart einer ionischen Flüssigkeit ist beispielsweise in EP-A 0 776 880 beschrieben.

Bei der Heck-Reaktion werden Arylhalogenide, Phenole, Aniline, Benzoesäureanhydride, Benzoesäurechloride oder vicinale Halogenolefine mit Olefinen an einem geeigneten Katalysator zu Styrolderivaten oder konjugierten Olefinen umgesetzt. Als geeignete Katalysatoren seien Palladium-Komplexe, insbesondere mit Phosphinoder N-heterocyclischen Carbenliganden genannt. Die Heck-Reaktion erfolgt im Allgemeinen bei einer Temperatur von 0 bis 200°C und einem Druck von Atmosphärendruck bis 32,5 MPa abs. Vorteilhafterweise können basische Verbindungen wie etwa Triethylamin, Cäsiumcarbonat oder Natriumhydrogencarbonat zugegeben werden. Die Heck-Reaktion von Arylhalogeniden oder Benzoesäureanhydrid mit Olefinen in Gegenwart einer ionischen Flüssigkeit ist beispielsweise in A.J. Carmichael et al., Org. Lett. 1999, Vol. 1, No. 7, Seite 997 bis 1000 beschrieben.

Bei der Hydrodimerisierung werden konjugierte Diene, wie beispielsweise 1,3-Butadien, in Gegenwart eines geeigneten Katalysators und Wasser zu Dienolen dimerisiert. So kann beispielsweise aus 1,3-Butadien 2,7-Octadienol gewonnen werden. Als geeignete Katalysatoren seien beispielhaft Verbindungen des Palladiums genannt. Die Hydrodimerisierung erfolgt im Allgemeinen bei einer Temperatur von 20 bis 200°C und einem Druck von Atmosphärendruck bis 20 MPa abs. Gegebenenfalls ist es von Vorteil, unter Kohlendioxid-Druck zu arbeiten. Die Hydrodimerisierung von 1,3-Butadien in Gegenwart einer ionischen Flüssigkeit ist beispielsweise in J.E.L. Dullius et al., Organometallics 17, 1998, Seite 815 bis 819 beschrieben.

Bei der Telomerisation werden konjugierte Diene, wie beispielsweise 1,3-Butadien, durch Umsetzung an einer Brönsted-Säure an einem geeigneten Katalysator zu den korrespondierenden funktionalisierten Dienen dimerisiert. So erhält man beispielsweise aus 1,3-Butadien 1,7- und 2,7-Octadienylderivate. Als Brönsted-Säuren sind beispielsweise Wasser, Alkohole oder Amine geeignet. Als geeignete Katalysatoren seien beispielhaft Verbindungen des Palladiums wie PdX₂ oder L₂PdX₂, wobei X für Chlor, Brom oder Acetat und L für einen Zweielektronen-Donor wie Phosphin oder Amin steht, genannt. Die Telomerisation erfolgt im Allgemeinen bei einer Temperatur von 20 bis 200°C und einem Druck von Atmosphärendruck bis 20 MPa abs. Gegebenenfalls ist es von Vorteil, in Gegenwart einer Base wie einem Alkalihydroxid oder einem tertiären Amin sowie unter Kohlendioxid-Druck zu arbeiten. Des Weiteren ist es gegebenenfalls vorteilhaft, ein unpolares Co-Solvens, wie beispielsweise Heptan, zuzusetzen. Die Telomerisation von 1,3-Butadien in Gegenwart einer ionischen Flüssigkeit ist beispielsweise in EP-A 1 201 634 beschrieben.

Bei der Trost-Tsuji-Kupplung werden Allylalkohole, Allylhalogenide oder Allylcarboxylate an einem geeigneten Katalysator mit einer Verbindung, welche ein acides, abspaltbares Wasserstoffatom enthält, wie etwa einem Amin, einem Malonsäurederivat oder einem 1,3-Diketon, unter Substitution des Alkohol-, Halogenid- oder Carboxylatrests umgesetzt. Als geeignete Katalysatoren seien beispielhaft Verbindungen des Palladiums genannt. Gegebenenfalls ist es von Vorteil, in Gegenwart einer Base zu arbeiten. Die Trost-Tsuji-Kupplung erfolgt im Allgemeinen bei einer Temperatur von 20 bis 200°C und einem Druck von Atmosphärendruck bis 20 MPa abs. Die Trost-Tsuji-Kupplung in Gegenwart einer ionischen Flüssigkeit ist beispielsweise in W. Chen et al., Chem. Commun. 1999, Seite 1247 bis 1248 beschrieben.

Bei der Suzuki-Kupplung werden Aryl- oder Alkylboronsäuren oder - ester mit Arylhalogeniden, Arylphenolestern, Anilinen, Diazoniumsalzen oder Arylcarbonsäurederivaten, wie Estern, Anhydriden oder Säurechloriden, in Gegenwart eines geeigneten Katalysators zu unsymmetrischen oder symmetrischen Biarylen umgesetzt. Als geeignete Katalysatoren seien beispielhaft Verbindungen des Palladiums oder Nickels, gegebenenfalls mit Liganden, wie etwa Trialkyl- oder Triarylphosphinen oder N-heterocyclischen Carbenen, genannt. Gegebenenfalls ist es vorteilhaft, ein unpolares Co-Solvens, wie beispielsweise Toluol, Dioxan oder Tetrahydrofuran, zuzusetzen. Des Weiteren ist es vorteilhaft, zur Aktivierung des Katalysators Fluorid oder eine Base zuzuführen. Die Suzuki-Kupplung erfolgt im Allgemeinen bei einer Temperatur von 20 bis 200°C und einem Druck von Atmosphärendruck bis 20 MPa abs. Die Suzuki-Kupplung ist beispielsweise in J. Hassan et al., Chem. Rev. 102, 2002, Seite 1359 bis 1469 beschrieben.

Bei der Kumada-Kupplung werden Aryl- oder Alkyl-magnesiumhalogenide (Grignard-Verbindungen) und bei der Negishi-Kupplung Aryloder Alkyl-zinkhalogenide mit Arylhalogeniden, Arylphenolestern, Anilinen oder Diazoniumsalzen in Gegenwart eines geeigneten Katalysators zu unsymmetrischen oder symmetrischen Biarylen umgesetzt. Als geeignete Katalysatoren seien beispielhaft Verbindungen des Palladiums oder Nickels, gegebenenfalls mit Liganden, wie etwa Trialkyl- oder Triarylphosphinen oder N-heterocyclischen Carbenen, genannt. Gegebenenfalls ist es vorteilhaft, ein unpolares Co-Solvens, wie beispielsweise Toluol, Dioxan oder Tetrahydrofuran, zuzusetzen. Die Kumada-Kupplung und die Negishi-Kupplung erfolgen im Allgemeinen bei einer Temperatur von 20 bis 200°C und einem Druck von Atmosphärendruck bis 20 MPa abs. Die Die Kumada-Kupplung und die Negishi-Kupplung sind beispielsweise in J. Hassan et al., Chem. Rev. 102, 2002, Seite 1359 bis 1469 beschrieben.

Bei der Stille-Kupplung werden Aryl- oder Alkylstannane mit Arylhalogeniden, Arylphenolestern, Anilinen, Diazoniumsalzen oder Arylcarbonsäurederivaten, wie Estern, Anhydriden oder Säurechloriden, in Gegenwart eines geeigneten Katalysators zu unsymmetrischen oder symmetrischen Biarylen umgesetzt. Als geeignete Katalysatoren seien beispielhaft Verbindungen des Palladiums oder Nickels, gegebenenfalls mit Liganden, wie etwa Trialkyl- oder Triarylphosphinen oder N-heterocyclischen Carbenen, genannt. Gegebenenfalls ist es vorteilhaft, ein unpolares Co-Solvens, wie beispielsweise Toluol, Dioxan, Tetrahydrofuran oder Dimethylformamid, zuzusetzen. Des Weiteren ist es vorteilhaft, zur Aktivierung des Katalysators eine Kupfer-haltige Verbindung, wie beispielsweise CuI oder CuO, zuzuführen. Die Stille-Kupplung erfolgt im Allgemeinen bei einer Temperatur von 20 bis 200°C und einem Druck von Atmosphärendruck bis 20 MPa abs. Die Stille-Kupplung ist beispielsweise in J. Hassan et al., Chem. Rev. 102, 2002, Seite 1359 bis 1469 beschrieben.

Bei der Sonogashira-Reaktion werden terminale Alkine mit Arylhalogeniden, Arylphenolestern, Anilinen, Diazoniumsalzen, vicinalen Halogenolefinen oder Arylcarbonsäurederivaten, wie Estern, Anhydriden oder Säurechloriden, in Gegenwart eines geeigneten Katalysators zu substituierten Alkinen umgesetzt. Als geeignete Katalysatoren seien beispielhaft Verbindungen des Palladiums oder Nikkels, gegebenenfalls mit Liganden, wie etwa Trialkyl- oder Triarylphosphinen oder N-heterocyclischen Carbenen, genannt. Gegebenenfalls ist es vorteilhaft, ein unpolares Co-Solvens, wie beispielsweise Toluol, Dioxan oder Tetrahydrofuran, zuzusetzen. Des Weiteren ist es vorteilhaft, zur Aktivierung des Katalysators eine Kupfer-haltige Verbindung, wie beispielsweise CuI, zuzuführen. Ferner ist es gegebenenfalls vorteilhaft, eine Base, wie beispielsweise Triethylamin, zuzusetzen. Die Sonogashira-Reaktion erfolgt im Allgemeinen bei einer Temperatur von 20 bis 200°C und einem Druck von Atmosphärendruck bis 20 MPa abs. Die Sonogashira-Reaktion ist beispielsweise in J. Hassan et al., Chem. Rev. 102, 2002, Seite 1359 bis 1469 beschrieben.

Bei der Carbonylierung werden Olefine, Alkohole oder Halogenverbindungen in Gegenwart eines geeigneten Katalysators mit Kohlenmonoxid und einer nukleophilen Verbindung, wie beispielsweise Wasser, Alkoholen oder Aminen zu den entsprechenden Carbonsäurederivaten umgesetzt. Als geeignete Katalysatoren seien beispielhaft Verbindungen des Palladiums, Iridiums oder Rhodiums, gegebenenfalls mit Liganden , wie etwa Trialkyl- oder Triarylphosphinen oder N-heterocyclischen Carbenen, insbesondere chelatisierende Liganden genannt. Die Carbonylierung erfolgt im Allgemeinen bei einer Temperatur von 0 bis 200°C und einem Druck von Atmosphärendruck bis 32,5 MPa abs.

Bei der Isomerisierung werden in Gegenwart eines geeigneten Katalysators Doppelbindungen innerhalb eines Kohlenstoffgerüsts verschoben. Als geeignete Katalysatoren seien beispielhaft Verbindungen des Rutheniums, Rhodiums, Kupfers oder Scandiums sowie Lewis- und Brönstedsäuren genannt.

Unter Umlagerungen sind beispielsweise die Cope-Umlagerung und die Beckmann-Umlagerung zu verstehen. Sie werden im Allgemeinen durch Lewissäuren, beispielsweise durch Scandium- oder Titan-Verbindungen katalysiert.

Bei der Diels-Alder-Reaktion wird ein Dien in Gegenwart eines geeigneten Katalysators mit einem Dienophil, beispielsweise einem Olefin, einem Alkin oder einem Carbonyl, unter Bildung eines Sechsrings umgesetzt. Als geeignete Katalysatoren werden im Allgemeinen Lewissäuren, beispielsweise Scandium- oder Titan-Verbindungen, eingesetzt. Die Diels-Alder-Reaktion in ionischen Flüssigkeiten ist beispielsweise in F. Zulfigar et al., Green Chem. 2, 2000, Seite 137 bis 139 beschrieben.

Bei der Metathese-Reaktion werden zwei Olefine in Gegenwart eines geeigneten Katalysators unter Bildung zweier anderer Olefine umgesetzt. Als geeignete Katalysatoren seien beispielhaft Verbindungen des Rutheniums, Osmiums, Rheniums, Molybdäns, Wolframs oder Titans genannt. Als Zusätze werden im Allgemeinen Tetramethylzinn, Alkylaluminiumchloride, Kupfer(I)-chlorid oder Pyridine zugegeben. Die Metathese-Reaktion ist beispielsweise in K.J. Ivin et al., Olefin Metathesis and Metathesis Polymerization, Academic Press, 1997 beschrieben.

Bei der Hydrocyanierung werden Olefine in Gegenwart geeigneter Katalysatoren mit Cyanwasserstoff zu Cyanoalkanen umgesetzt. Als geeignete Katalysatoren seien beispielhaft Verbindungen des Nikkels genannt.

Bei der Oligomerisation werden Olefine oder Epoxide in Gegenwart eines geeigneten Katalysators zu den entsprechenden höhermolekularen Verbindungen oligomerisiert. Werden Dimere gebildet, so spricht man beispielsweise von einer Dimerisierung, werden Trimere gebildet, so spricht man von einer Trimerisierung. Als geeignete Katalysatoren seien beispielhaft Verbindungen des Nickels, Aluminiums oder Lewissäuren, wie etwa Scandium- oder Titan-Verbindungen, genannt. Des Weiteren sind für die Oligomerisation von Epoxiden auch Lewis-Basen, Brönsted-Säuren, wie etwa Phosphorsäure oder Schwefelsäure, oder Brönsted-Basen, wie etwa Kaliumhydroxid, geeignet.

Bei der Polymerisation werden Olefine, Epoxide, Lactame, Lactone oder Alkine in Gegenwart eines geeigneten Katalysators zu polymeren Verbindungen umgesetzt. Als geeignete Katalysatoren seien beispielhaft Verbindungen des Titans, Zirkons, Hafniums, Nickels, Eisens, Palladiums, Aluminiums, Lithiums sowie für cyclische Olefine auch des Rutheniums, Osmiums, Wolframs, Molybdäns oder Titans genannt. Die Polymerisation ist beispielsweise in G. Odian, Principles of Polymerization, Wiley & Sons 1991 beschrieben. Die oben genannten chemischen Reaktionen können beim erfindungsgemäßen Verfahren diskontinuierlich, halbkontinuierlich oder kontinuierlich durchgeführt werden.

Durch das besondere Verfahren zur Herstellung des einzusetzenden 1,3-substituierten Imidazoliumsalzes ist es möglich, beim erfindungsgemäßen Verfahren zur Durchführung der chemischen Reaktionen einen besonders niedrigen Gehalt an Chlorid-, Bromid- und Iodidionen zu gewährleisten. Im Allgemeinen beträgt die Konzentration an Chlorid-, Bromid- und Iodidionen in der Flüssigphase in Summe ≤100 Gew.-ppm, bevorzugt ≤50 Gew.-ppm und besonders bevorzugt ≤20 Gew.-ppm. Dieser niedrige Gehalt an Chlorid-, Bromid- und Iodidionen ist von ganz besonderem Vorteil, da gerade diese Halogenidionen bei den chemischen Umsetzungen die Aktivität des Katalysators deutlich mindern. Die folgenden Literaturzitate sollen dies belegen:

Aus P. Kraus et al., Journal of Enzyme Inhibition 10, 1996, Seiten 125 bis 133 ist beispielsweise bekannt, dass bereits 50 bis 100 mMol/L Chlorid die Gamma-Aminobuttersäure Aminotransferase aus den Mitochondrien des Gehirns und der Leber von Ratten um 10 bis 30% inhibieren. Des Weiteren ist beispielsweise aus B.G. Bolscher et al., Biochemica et Biophysica Acta 788(1), 1984, Seiten 1 bis 10 bekannt, dass das Chloridion an der menschlichen Myeloperoxidase kompetitiv zu Wasserstoffperoxid am aktiven Zentrum des Enzyms bindet und somit inhibierend wirkt. Aus M.C. Simpson et al., Coordination Chem. Rev. 155, 1996, Seite 163 bis 207 ist beispielsweise bekannt, dass bei der Hydrierung von Olefinen in Gegenwart eines [Rh(cod)₂][BF₄]-Katalysators, wobei cod für Cyclooctadien steht, ein Austausch des nicht-koordinierenden [BF₄]-Anions durch ein koordinierendes Halogenid-Anion die Aktivität des Katalysator deutlich herabsetzt. Der störende Einfluss von Chloridionen bei der Suzuki-Kupplung ist beispielsweise in P.J. Dyson et al., Electrochemical Society Proceedings 99, 2000, Seiten 161 bis 168 beschrieben. Aus dem Bereich der Olefin-Metathese ist beispielsweise nach D. Semeril et al., Chem. Commun., 2002, Seiten 146 bis 147 bekannt, dass die Aktivität kationischer Ruthenium-Komplexe, wie etwa [RuCl(PCy₃)(p-cymol)=C=C=CPh₂][OTf], wobei Cy für Cyclohexyl, p-cymol für p-Methylcumol, Ph für Phenyl und [OTf]- für Trifluormethylsulfonat (Triflat) steht, durch den Austausch des [OTf]⁻-Anions gegen ein Halogenid-Anion sinkt. In M. Shibaski et al., J. Organomet. Chem. 576, 1999, Seite 1 ist beschrieben, dass bei der asymmetrischen Heck-Reaktion zur Erreichung hoher Enantiomerenüberschüsse Halogenidionen mit Abfangreagenzien wie etwa Ag₃PO4 oder Ag(OTf), wobei [OTf]⁻ für Trifluormethylsulfonat (Triflat) steht, aus der Reaktionsmischung entfernt werden müssen. Aus L.E. Overmann et al., Angew. Chem. 109, 1997, Seite 536 ist beispielsweise bekannt, dass bei der Heck-Reaktion in einigen Fällen durch die Gegenwart von Halogenidionen die Stereochemie am asymmetrisch kooridinierten Kohlenstoffzentrum invertiert werden kann. Nach J.E. Bäckvall, Acc. Chem. Res. 16, 1983, Seite 335 tritt in der Palladium-katalysierten 1,4-Funktionalisierung von 1,3-Dienen durch die Anwesenheit von Halogenidionen eine Änderung der Stereoselektivität auf. Bei der homogenen Olefin-Polymerisation in Gegenwart von Metallocenen von Metallen der 4. Gruppe des Periodensystems oder in Gegenwart von Schiffbasen-Komplexen mit Metallen der 8. bis 10. Gruppe des Periodensystems sind diese nur als kationische Komplexe aktiv, welche durch die Anwesenheit von Halogenidionen gequenscht werden und somit die Katalysatoren deaktivieren. Übersichten hierzu finden sich in R.F. Jorden, Adv. Organomet. Chem. 32, 1991, Seite 325, in H.H. Brintzinger et al., Angew. Chem. 107, 1995, Seite 1255 und in S.D. Ittel et al., Chem. Rev. 100, 2000, Seite 1169.

In einer bevorzugten Ausführungsform zur Herstellung des 1,3-substituierten Imidazoliumsalzes (I) gibt man das 1-substituierte Imidazol (II), den Kohlensäurediester (III), Lösungsmittel, bevorzugt Alkohol und gegebenenfalls den Katalysator, bevorzugt Na⁺ oder K⁺ in Verbindung mit einem geeigneten Phasentransfer-Komplexbildner wie beispielsweise einem Kronenether, in einen geeigneten Reaktionsapparat, bevorzugt einem Rührkessel und erwärmt auf die gewünschte Reaktionstemperatur. Infolge der Flüchtigkeit der niedermolekularen Kohlensäurediester ist es vorteilhaft, die Reaktion unter Druck durchzuführen. Nach erfolgter Umsetzung kühlt man das Reaktionsgemisch ab und versetzt mit der gewünschten Protonensäure (IV) oder deren Salz in Form einer wässrigen Lösung. Anschließend trennt man beide Phasen und wiederholt gegebenenfalls die Zugabe der Protonensäure (IV) oder deren Salzes zu der das 1,3-substituierte Imidazoliumsalz (I) enthaltenden Phase. Die das 1,3-substituierte Imidazoliumsalz (I) enthaltenden Phasen werden im Allgemeinen vereint und gegebenenfalls zur weiteren Abreicherung von Verunreinigungen einer Extraktion unterzogen. Gegebenenfalls wird die das 1,3-substituierte Imidazoliumsalz (I) enthaltende Phase filtriert, säulenchromatographisch gereinigt und/oder mit Aktivkohle behandelt. Die erhaltene Phase wird dann im Allgemeinen destillativ von gegebenenfalls noch vorhandenen Lösungsmittel befreit. Der verbleibende Rückstand kann direkt weiter verwendet, getrocknet-, aus einem geeigneten Lösungsmittel kristallisiert oder die Schmelze geschuppt werden. Eine weitere Reinigung kann zum Beispiel durch Zonenschmelze oder Tieftemperatur-Kristallisation erfolgen.

In einer bevorzugten Ausführungsform zur diskontinuierlichen Durchführung einer chemischen Reaktion in der Flüssigphase legt man im Allgemeinen das 1,3-substituierte Imidazoliumsalz, gegebenenfalls den Katalysator und die Edukte sowie gegebenenfalls Lösungsmittel vor und bringt das Gemisch auf die gewünschten Reaktionsbedingungen. Nach erfolgter Umsetzung trennt man das erhaltene Reaktionsprodukt beispielsweise durch Phasentrennung und/oder Extraktion vom Reaktionsgemisch ab und arbeitet dieses nach den an sich bekannten Verfahren auf.

In einer bevorzugten Ausführungsform zur halkontinuierlichen Durchführung einer chemischen Reaktion in der Flüssigphase legt man im Allgemeinen das 1,3-substituierte Imidazoliumsalz, gegebenenfalls den Katalysator und gegebenenfalls ein Edukt sowie gegebenenfalls ein Co-Lösungsmittel vor, bringt das Gemisch auf die gewünschten Reaktionsbedingungen und führt das andere oder die anderen Edukte über einen gewissen Zeitraum hin zu. Die Abtrennung und Isolation des gewünschten Produkts erfolgt im Allgemeinen wie bereits zuvor beschrieben.

In einer bevorzugten Ausführungsform zur kontinuierlichen Durchführung einer chemischen Reaktion in der Flüssigphase führt man im Allgemeinen alle Edukte sowie den Katalysator, falls dieser ausgetragen oder verbraucht wird, gegebenenfalls Lösungsmittel und gegebenenfalls das 1,3-substituierte Imidazoliumsalz (I), falls dieses ausgetragen wird, kontinuierlich zu. Je nach Flüchtigkeit des gewünschten Produkts ist es gegebenenfalls vorteilhaft, dieses kontinuierlich durch Verdampfung aus dem Reaktionsgemisch zu entfernen und wie gewohnt aufzuarbeiten. Alternativ und bei weniger flüchtigen Produkten kann man das gewünschte Produkt natürlich auch wie oben beschrieben abtrennen und isolieren.

Das erfindungsgemäße Verfahren ermöglicht die Durchführung chemischer Reaktionen in der Flüssigphase durch Umsetzung der Edukte in Gegenwart eines 1,3-substituierten Imidazoliumsalzes und gegebenenfalls eines geeigneten Katalysators unter Erzielung eines hohen Umsatzes, einer hohen Selektivität, einer hohen Ausbeute und einer hohen Raum-Zeit-Ausbeute, bei dem das einzusetzende 1,3-substituierte Imidazoliumsalz in einfacher Art und Weise unter Vermeidung des Einsatzes toxischer, karzinogener und ätzender Einsatzstoffe und ohne aufwändige Reinigung praktisch chlorid-, bromid- und iodidfrei zugänglich ist. Der besonders niedrige Gehalt an Chlorid-, Bromid- und Iodidionen ist beim erfindungsgemäβen Verfahren von ganz besonderem Vorteil, da gerade diese Halogenidionen bei den chemischen Umsetzungen die Aktivität des Katalysators deutlich mindern.

Des Weiteren ist Gegenstand der Erfindung die Verwendung des erfindungsgemäß hergestellten 1,3-substituierten Imidazoliumsalzes (I) als ionisches Lösungsmittel in der erfindungsgemäßen Durchführung chemischer Reaktionen in der Flüssigphase in Gegenwart eines Katalysators.

### Beispiele

### Beispiel 1: Herstellung von 1-Butyl-3-methylimidazolium-hexafluorophosphat

In einem Autoklaven wurden 0,25 Mol 1-Butylimidazol (Fa. Aldrich) und 0,375 Mol Dimethylcarbonal in 64 g Methanol 7 Stunden bei 135°C gerührt. Der abgekühlte Austrag wurde mit 0,5 Mol Natriumhexaflourophosphat und 150 ml Aceton versetzt. Es wurde 2 Stunden bei 25°C gerührt und ausgefallene Feststoff abgetrennt. Das Filtrat wurde in 200 ml Essigester aufgenommen, zweimal mit je 10 ml Wasser gewaschen und dann im Vakuum von allen Lösungsmittelresten und nicht-umgesetzten Edukten befreit. Es wurden 0,13 Mol 1-Butyl-3-methylimdazolium-hexaflourophosphat erhalten (52% d.Th.).

### Beispiel 2: Herstellung von 1-Butyl-3-methylimdazolium-tetraflouroborat

In einem Autoklaven wurden 0,5 Mol 1-Butylimidazol (Fa. Aldrich) und 0,6 Mol Dimethylcarbonal in 50 g Methanol 12 Stunden bei 135°C gerührt. Der abgekühlte Austrag wurde im Vakuum vom Lösungsmittel und nicht-umgesetzten Edukten befreit. Es wurden 0,5 Mol Tetraflourborsäure in Diethylether (Fa. Aldrich) zugegeben, 2 Stunden gerührt und die Reaktionsmischung dann im Vakuum von Lösungsmittelresten befreit Es wurden 0,34 Mol 1-Butyl-3-methylimdazoliumtetrafluoroborat, im Folgenden vereinfacht als [BMIM] [BF_{4]} bezeichnet, erhalten (68 % d.Th.).

### Beispiel 3: Herstellung von 1-Decyl-3-methylimidazolium-tetrafluoroborat

In einem Autoklaven wurden 0,25 Mol 1-Decylimidazol, welches durch die Radziszewski-Reaktion aus Glyoxal, Formaldehyd, Ammoniak und 1-Decylamin zugänglich ist (siehe Ullmann's Encyclopedia of Industrial Chemistry, 6^{th} edition, 2000 electronic release, Chapter "IMIDAZOLE AND DERIVATIVES - Production), und 0,3 Mol Dimethylcarbonat in 100 ml Methanol zusammen mit 1,5 g Kaliumcarbonat und 0,1 g 18-Krone-6 12 Stunden auf 135°C erhitzt. Nach dem Abkühlen wurde der Reaktionsaustrag filtriert und mit 150 ml 50%iger wässriger Tetrafluoroborsäure (Fa. Acros) versetzt. Nun wurde für eine Stunde unter Rühren auf 50°C erwärmt und anschlieβend die wässrige Phase abgetrennt. Die verbleibende organische Phase wurde erneut mit 100 ml 50 %iger wässriger Tetrafluoroborsäure versetzt, für eine weitere Stunde unter Rühren auf 50°C erwärmt und die wässrige Phase abgetrennt. Der organische Rückstand wurde nun zweimal mit Cyclohexan extrahiert und danach im Vakuum bei 80°C von Lösungsmittelresten befreit. Es wurden 0,16 Mol 1-Decyl-3-methylimidazolium-tetrafluoroborat erhalten, was einer Ausbeute von 64 % entspricht.

### Beispiel 4: Herstellung von 1-Butyl-3-methylimidazolium-tetrafluoroborat [BMIM] [BF₄] mit verschiedenen Chloridgehalten

Das in Beispiel 2 hergestellte [BMIM] [BF₄] wies laut Elementaranalyse einen Chloridgehalt von 20 ppm auf.

[BMIM] [BF₄] mit 50 ppm und 260 ppm Chloridgehalt wurden hergestellt durch Mischen von 2,5 mL [BMIM] [BF₄] mit 20 ppm Chloridgehalt aus Beispiel 2 und einer entsprechenden Menge an [BMIM]Cl (1-Butyl-3-methylimidazolium-chlorid). Die Bestimmung des Chlorid-gehalts erfolgte ebenfalls durch Elementaranalyse.

### Beispiel 5: Suzuki-Reaktion

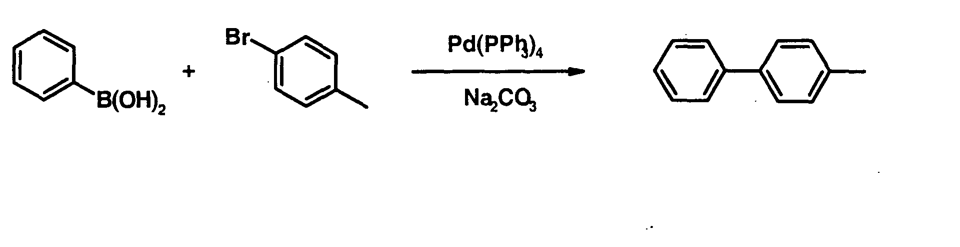

86,6 mg Pd(PPh₃)₄ (Fa. Aldrich) wurden in jeweils 5 mL [BMIM] [BF₄] mit verschiedenen Chloridgehalten aus Beispiel 4 unter Argon vorgelegt. Es wurden 428 mg Bromtoluol (Fa. Aldrich) zugegeben. Die Mischung wurde 10 Minuten bei 80°C gerührt, wobei sich eine klare, gelb-orange Lösung ergab. Anschließend wurden 0,2 mL entgastes Wasser und 560 mg Natriumcarbonat (Fa. Aldrich) hinzugefügt. Innerhalb von 15 Minuten wurden schließlich 335 mg Phenylboronsäure (Fa. Aldrich) zugegeben. Die Reaktion wurde 2 Stunden bei 80°C weitergerührt, bevor nach Abkühlen auf Raumtemperatur die [BMIM] [BF₄] enthaltende Phase mit Toluol extrahiert wurde. Die erhaltene organische Phase wurde per GC/MS analysiert. Die Ergebnisse sind in Tabelle 1 dargestellt.

**Tabelle 1:**

| Ergebnisse aus Beispiel 5 | | |
|---|---|---|
| [BMIM] [BF_{4]} mit | 4-Methylbiphenyl [GC-Flächen-%] | Biphenyl [GC-Flächen-%] |
| 20 ppm Chloridgehalt | 83 | 6 |
| 50 ppm Chloridgehalt | 81 | 7 |
| 260 ppm Chloridgehalt | 34 | 5 |

Die Versuchsreihe zeigt, dass die Ausbeute an Wertprodukt 4-Methylbiphenyl mit sinkendem Chloridgehalt steigt. So wird bei einem Chloridgehalt von 50 ppm im [BMIM] [BF₄] eine mehr als doppelt so hohe Ausbeute erreicht als bei einem Chloridgehalt von 260 ppm.

### Beispiel 6: Diels-Alder-Reaktion

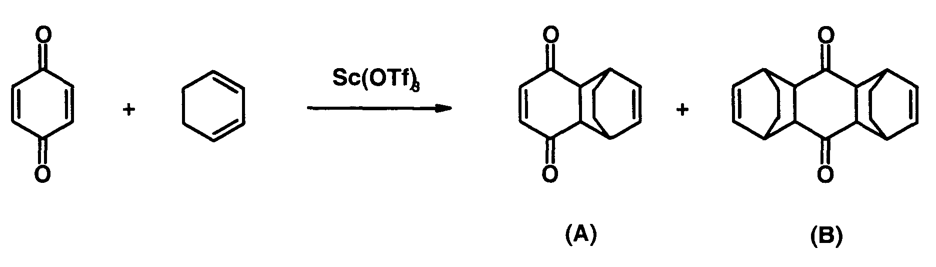

540 mg Benzochinon (Fa. Aldrich) wurden in jeweils 1130 mg [BMIM] [BF_{4]} mit verschiedenen Chloridgehalten aus Beispiel 4 unter Argon vorgelegt. Es wurden 1200 mg 1,3-Cyclohexadien (Fa. Aldrich) und anschließend 5 mg Sc(OTf)₃ (Fa. Aldrich) hinzugefügt. Die Mischung wurde 1 Stunde bei Raumtemperatur gerührt und anschließend mit Toluol extrahiert. Der Katalysator blieb in der [BMIM][BF₄] enthaltenden Phase zurück. Da die Reaktion auch unkatalysiert langsam fortschreitet, wurde die Toluolphase sofort per GC/MS analysiert. Die Ergebnisse sind in Tabelle 2 dargestellt.

**Tabelle 2:**

| Ergebnisse aus Beispiel 6 | | | |
|---|---|---|---|
| [BMIM][BF₄] mit | Umsatz [%] | Monoprodukt (A) [GC-Flächen-%] | Diprodukt (B) [GC-Flächen-%] |
| 20 ppm Chloridgehalt | 97 | 86 | 11 |
| 50 ppm Chloridgehalt | 95 | 88 | 7 |
| 260 ppm Chloridgehalt | 73 | 73 | 0 |

Die Versuchsreige zeigt, dass der Umsatz an Benzochinon mit abnehmendem Chloridgehalt ansteigt. Die Aktivität des Katalysators wird im betrachteten Fall sogar so hoch, dass bei sehr niedrigen Chloridgehalten von 20 ppm und 50 ppm sogar die zweifache Diels-Alder Reaktion zum Diprodukt (B) stattfindet.

## Patentansprüche

1. Verfahren zur Durchführung chemischer Reaktionen in der Flüssigphase durch Umsetzung der Edukte in Gegenwart
(a) eines Katalysators; und
(b) eines 1,3-substituierten Imidazöliumsalzes der allgemeinen Formel (I) 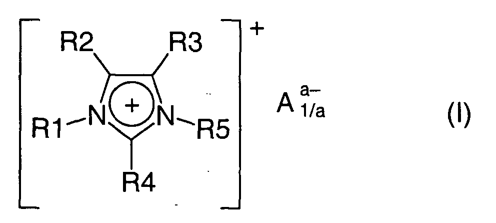 in der
* die Reste R², R³ und R⁴ unabhängig voneinander Wasserstoff, Halogen oder eine funktionelle Gruppe und die Reste R¹, R², R³, R⁴ und R⁵ unabhängig voneinander einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R⁴ und R¹, R¹ und R² sowie R² und R³ auch miteinander verbunden sein können;
und
* A^{a-} für das Anion einer Protonensäure HₐA (IV) steht, wobei a eine ganze, positive Zahl ist und den Ladungszustand des Anions wiedergibt, und A^{a-} für
Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat;
tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/ oder durch eine oder mehrere funktionelle Gruppen oder Halogen substituiert sein kann, stehen;
organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
(Fluoralkyl)fluorphosphat der allgemeinen Formel (Vd) [PFₓ(C_{y}F_{2y+1-z}H_{z})₆₋ₓ]⁻, wobei 1 ≤ x ≤6, 1 ≤ y ≤8 und 0 ≤ z ≤ 2y+1; oder
Imid der allgemeinen Formeln (Ve) [R^{g}-SO₂-N-SO₂-R^{h}]⁻, (Vf) [Rⁱ-SO₂-N-CO-R^{j}]⁻ oder (Vg) [R^{k}-CO-N-CO-R¹]⁻, wobei R^{g} bis R¹ unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
steht,
und welches einen Schmelzpunkt bei Normaldruck von ≤200°C aufweist,
**dadurch gekennzeichnet, dass** man ein 1,3-substituiertes Imidazoliumsalz (I) einsetzt, welches durch
(i) Umsetzung eines 1-substituierten Imidazols der allgemeinen Formel (II) 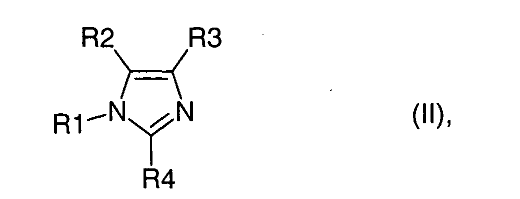 in der die Reste R¹ bis R⁴ die oben genannte Bedeutung besitzen, mit einem Kohlensäurediester der allgemeinen Formel (III) 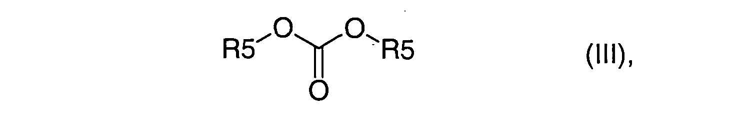 in der der Rest R⁵ die oben genannte Bedeutung besitzt, bei einer Temperatur im Bereich von 10 bis 350°C (283 bis 623 K) und einem Druck im Bereich von 0,01 bis 5 MPa abs; und
(ii) Umsetzung des Umsetzungsprodukts aus Schritt (i) mit der gewünschten Protonensäure HₐA (IV) oder deren Salzen bei einer Temperatur im Bereich von -80 bis 200°C (193 bis 473 K) und einem Druck im Bereich von 0,001 bis 1 MPa abs zum 1,3-substituierten Imidazoliumsalz (I) ;
hergestellt wurde.

2. Verfahren nach Anspruch 1, **dadurch gekennzeichnet, dass** man ein 1,3-substituiertes Imidazoliumsalz (I) einsetzt, bei dem die Reste R², R³ und R⁴ unabhängig voneinander Wasserstoff, Fluor, Chlor, Brom, Phenyl, C₆F₍₅₋ₐ₎ Hₐ mit 0 ≤a ≤5 oder eine unverzweigte oder verzweigte C₁- bis C₁₂-Alkylgruppe und die Reste R¹ und R⁵ unabhängig voneinander eine unverzweigte oder verzweigte C₁- bis C₁₂-Alkylgruppe bedeuten.

3. Verfahren nach den Ansprüchen 1 bis 2, **dadurch gekennzeichnet, dass** man ein 1,3-substituiertes Imidazoliumsalz (I) einsetzt, bei dem das Anion A^{a-} für Tetrafluoroborat, Hexafluorophosphat, Trifluormethansulfonat, Methansulfonat, Formiat, Acetat, Mandelat, Nitrat, Nitrit oder Trifluoracetat steht.

4. Verfahren nach den Ansprüchen 1 bis 3, **dadurch gekennzeichnet, dass** man ein 1,3-substituiertes Imidäzoliumsalz (I) einsetzt, bei dessen Herstellung in Schritt (i) das Molverhältnis zwischen dem 1-substituierten Imidazol (II) und dem Kohlensäurediester (III) 0,2 bis 10 betrug.

5. Verfahren nach den Ansprüchen 1 bis 4, **dadurch gekennzeichnet, dass** man ein 1,3-substituiertes Imidazoliumsalz (I) einsetzt, bei dessen Herstellung in Schritt (i) als Kohlensäurediester (III) Dimethylcarbonat oder Diethylcarbonat eingesetzt wurde.

6. Verfahren nach den Ansprüchen 1 bis 5, **dadurch gekennzeichnet, dass** man ein 1,3-substituiertes Imidazoliumsalz (I) einsetzt, bei dessen Herstellung in Schritt (ii) das Molverhältnis zwischen der gewünschten Protonensäure HₐA (IV) oder deren Salzen und dem in Schritt (i) eingesetztem 1-substituierten Imidazol (II) 1/ₐ · 0,2 bis 1/ₐ · 5 betrug.

7. Verfahren nach den Ansprüchen 1 bis 6, **dadurch gekennzeichnet, dass** man als Katalysator
(i) ein Metall der 3. bis 13. Gruppe des Periodensystems und/oder dessen Verbindungen, oder
(ii) einen Biokatalysator oder ein Biokatalysator enthaltendes System einsetzt.

8. Verfahren nach Anspruch 7, **dadurch gekennzeichnet, dass** man als Katalysator einen Ruthenium, Osmium, Cobalt, Rhodium, Iridium, Nickel, Palladium, Platin, Titan, Zirkon, Vanadium, Mangan und/oder Scandium enthaltenden Übergangsmetall-Komplex einsetzt.

9. Verfahren nach den Ansprüchen 1 bis 8, **dadurch gekennzeichnet, dass** man als chemische Reaktion eine Hydrierung, eine Oxidation, eine Hydroformylierung, eine Heck-Reaktion, eine Hydrodimerisierung, eine Telomerisation, eine Trost-Tsuji-Kupplung, eine Suzuki-Kupplung, eine Kumada-Kupplung, eine Negishi-Kupplung, eine Stille-Kupplung, eine Sonogashira-Reaktion, eine Carbonylierung, eine Isomerisierung, eine Umlagerung, eine Diels-Alder-Reaktion, eine Metathese-Reaktion, eine 1,4-Funktionalisierung von 1,3-Dienen, eine Hydrocyanierung, eine Oligomerisation oder eine Polymerisation durchführt.

10. Verfahren nach den Ansprüchen 1 bis 9, **dadurch gekennzeichnet, dass** die Konzentration an Chlorid-, Bromid- und Iodidionen in der Flüssigphase in Summe ≤50 Gew.-ppm beträgt.

11. Verwendung eines 1,3-substituierten Imidazoliumsalzes (I) wie in den Ansprüchen 1 bis 6 definiert als ionisches Lösungsmittel in der Durchführung chemischer Reaktionen in der Flüssigphase in Gegenwart eines Katalysators gemäß den Ansprüchen 1 bis 10.
